Europäisches Patentamt

European Patent Office

Office européen des brevets

(19)

(11) Numéro de publication: **0 092 085**
**B1**

(12) # FASCICULE DE BREVET EUROPEEN

(45) Date de publication du fascicule du brevet:
**12.03.86**

(21) Numéro de dépôt: **83103223.0**

(22) Date de dépôt: **31.03.83**

(51) Int. Cl.⁴: **C 11 B 1/10,** C 11 B 3/00,
A 23 L 1/36, A 61 K 35/78,
A 61 K 7/48 // A23L1/24,
A23C11/04

(54) **Compositions nutritives contenant des corps gras et procédé de préparation de ceux-ci.**

(30) Priorité: **16.04.82 CH 2314/82**

(43) Date de publication de la demande:
**26.10.83 Bulletin 83/43**

(45) Mention de la délivrance du brevet:
**12.03.86 Bulletin 86/11**

(84) Etats contractants désignés:
**AT BE CH DE FR IT LI LU NL SE**

(56) Documents cités:
**FR - A - 2 197 605**
**FR - A - 2 255 055**
**GB - A - 2 084 172**

(73) Titulaire: **SOCIETE DES PRODUITS NESTLE S.A., Case postale 353, CH-1800 Vevey (CH)**

(72) Inventeur: **Traitler, Helmut, Route de St-Légier 10a, CH-1800 Vevey (CH)**
Inventeur: **Winter, Heike, Route de St-Légier 25, CH-1800 Vevey (CH)**

**Description**

La présente invention concerne des compositions nutritives contenant des corps gras et un procédé de préparation de ceux-ci à partir de fruits du genre Ribes.

La plupart des huiles végétales contiennent les acides gras polyinsaturés linoléique (6,9-octadécadiénique) et alphalinolénique (9,12,15-octadécatriénique). Seules les huiles des graines de houblon (Humulus), de chanvre (Cannabis), de bourrache (Borage) et d'onagre (Oenothera) sont connues pour contenir l'acide gamma-linolénique (6,9,12-octadécatriénique), l'onagre en étant la seule source disponible par ailleurs à un prix élevé.

L'acide gamma-linolénique est un acide gras essentiel qui est métabolisé par l'organisme en prostaglandines via l'acide dihomo-gamma-linolénique et l'acide arachidonique (5,8,11,14-éicosatétraénique), qui est lui-même un constituant des membranes cellulaires tandis que l'acide alphalinolénique ne contribue pas de la même manière à ce processus métabolique. La conversion acide linoléique → acide gamma-linolénique dans les tissus est incomplète (4-20% comparé à 90-98% pour la transformation acide gamma-linolénique → acide arachidonique) et peut même être inexistante (p. ex. pour le chat) en cas d'absence ou d'inactivation de l'enzyme Δ-6-désaturase.

Un manque d'acides gras essentiels conduit en effet à une carence nutritionnelle affectant tous les processus métaboliques mentionnés plus haut et pouvant conduire à des désordres biochimiques ou à des lésions organiques (p. ex. troubles de la coagulation, lésions dermatologiques, troubles endocriniens, lésions myocardiques, troubles hépatiques, articulaires, neurologiques et mentaux). On voit donc l'intérêt que présente un apport d'acide gamma-linolénique pour la prévention ou pour le traitement de ces anomalies.

On a mentionné la possibilité d'utiliser les acides gamma-linolénique et arachidonique comme agents thérapeutiques et nutritionnels p. ex. dans les brevets français Nos. 2 197 605 et 1 603 383, l'acide gamma-linolénique étant d'origine synthétique ou extrait de l'huile de graine d'onagre (Oenothera) ou de bourrache officinale (Borage officinalis).

De plus, le brevet français No. 2 255 055 concerne des compositions cosmétiques ou pharmaceutiques à base d'huile de pépins de framboise dont on mentionne l'activité antiinflammatoire sans en indiquer la composition. L'huile étant extraite de préférence au chloroforme (solvant polaire), l'activité anti-inflammatoire est vraisemblablement due à la présence de composants mineurs. Une analyse de cette huile a par ailleurs montré qu'elle contenait environ 54% en poids d'acide linoléique et 30% d'acide alphalinolénique mais ne contenait pas d'acide gamma-linolénique.

Enfin, le brevet hongrois No. T 13 226 mentionne que l'addition d'un broyat ou de l'huile brute extraite à partir de pépins de paprika, de tomates ou de groseilles à des produits cosmétiques ou des huiles de table inhiberait leur oxydation. L'extraction telle qu'elle est prévue ne permet pas de séparer les colorants et les cires indésiderables dans les produits envisagés et la composition de l'extrait n'est pas mentionnée.

On a constaté de façon tout à fait surprenante que les huiles de pépins de fruits du genre Ribes contenaient un pourcentage appréciable, au moins 4% en poids d'acide gamma-linolénique. De plus ces pépins sont disponibles en grandes quantités dans les tourteaux provenant de pressage des jus de fruits, de la fabrication des confitures et gelées ou des moûts de fermentation provenant de la production des eaux-de-vie, liqueurs et schnaps, sous-produits qui étaient jusqu'à présent utilisés comme combustible ou comme fourrage.

L'invention concerne des compositions nutritives contenant de 1 à 80% en poids d'un corps gras extrait à partir de pépins de fruits du genre Ribes contenant au moins 4% en poids d'acide gamma-linolénique pratiquement dépourvu des composés odorants, des acides gras libres, des colorants et des cires de ces fruits.

Selon l'invention, le terme «composition nutritive» désigne des compositions cosmétiques, dermatologiques (cosmétique corrective) ou topiques (p. ex. ophtalmiques) ainsi que des aliments diététiques ou de supplémentation ou encore des compositions pharmaceutiques pour l'alimentation orale, entérale ou parentérale.

Selon l'invention, les corps gras entrant dans la composition nutritive proviennent en pratique du cassis (Ribes nigrum), des groseilles (Ribes rubrum), des groseilles à maquereau (Ribes ova-crispa ou grossularia) ou des fruits d'hybrides de ces espèces. On peut bien entendu utiliser un mélange de ces fruits.

Le contenu en lipides des sous-produits mentionnés ci-dessus est de 12 à 30% en poids suivant la matière première. La phase lipidique contient à son tour de 4 à 19% en poids d'acide gamma-linolénique.

A titre indicatif, l'huile des pépins de ces fruits est constituée de triglycérides des acides gras suivants, en poids:

| Acides gras | Cassis | Groseille | Groseille à maquereau |
|---|---|---|---|
| C 16:0 | 6 - 7% | 4 - 5 % | 7- 8% |
| C 18:0 | 1 - 2% | 1 - 2 % | 1- 1% |
| C 18:1 cis | 9 -10% | 14 -15 % | 15-16% |
| C 18:1 trans | 0,5% | 0,5- 1 % | 1- 2% |
| C 18:2 ω 6 | 47 -49% | 41 -42 % | 39-41% |
| C 18:3 ω 6 | 15 -19% | 4 - 5 % | 10-12% |
| C 18:3 ω 3 | 12 -14% | 29 -31 % | 19-20% |
| C 18:4 ω 3 | 3 - 4% | 2,5- 3,5% | 4- 5% |

L'huile de cassis qui est préférée à cause de sa teneur élévée en acide gamma-linolénique, contient en outre de 1 à 2% en poids de corp insaponifiables, tels que alcools aliphatiques, hydrocarbures, tocophérols, squalène, β-sitostérol, campestérol et Δ-7 stigmastérol. Sa densité est 0,9215 g/cm$^3$ (à 20°C) et sa viscosité 28,3 centipoise (à 20°C).

Une composition cosmétique ou dermatologique peut se présenter sous forme d'émulsion fluide eau dans l'huile ou huile dans l'eau (lait, lotion, shampoing, mousse à raser, etc...) ou d'émulsion plus consistante (crème, masque) et contenant généralement de 1 à 20% en poids du corps gras ci-dessus. Elle peut être essentiellement constituée d'une phase grasse (baume, huile de bain, etc...), contenant jusqu'à 80% du corps gras ci-dessus, ou encore avoir une phase aqueuse prédominante (produit de rinçage capillaire, etc...), Enfin, elle peut être essentiellement solide (produits de maquillage, fard à paupières, fond de teint, rouge à lèvres, etc...) et contenir de 1 à 30% du corps gras ci-dessus.

Sa phase grasse peur contenir outre le corps gras ci-dessus les huiles végétales, animales, minérales ou synthétiques, les cires, alcools à longue chaîne et polymères d'emploi courant en cosmétique.

Lorsqu'il s'agit d'émulsions, les compositions contiennen 1 à 20% en poids d'agents emulsionnants.

Les compositions peuvent en outre contenir des agents colorants, des parfums, des conservateurs, des pigments, des agents nacrants, des antioxydants et des charges.

Parmi les aliments diététiques ou de supplémentation envisagés, on peut citer les laits infantiles et plus particulièrement les laits maternisés, les sauces, les mayonnaises et les huiles pour salade. Dans ces produits, les corps gras sont dosés de telle sorte qu'ils fournissent l'équivalent de 0,35 à 2,5% en poids d'acide gamma-linolénique et représentent 2 à 15% en poids. Les corps gras seront avantageusement protégés de l'oxydation par addition des esters de l'acide ascorbique avec des acides gras p. ex. le palmitate d'ascorbyle.

On peut également envisager d'utiliser les corps gras ci-dessus en supplémentation animale, en particulier dans des aliments p. ex. des compositions carnées pour chats.

Enfin, les corps gras ci-dessus peuvent entrer dans la composition de médicaments de formulation adaptée au mode d'administration et administrés p. ex. sous forme de sirop ou de capsules ou d'émulsions isotoniques oculaires ou encore être des constituants de la phase grasse d'émulsions destinées à l'alimentation parentérale et représenter 5 à 80% en poids de la composition.

Les compositions pharmaceutiques selon l'invention sont stabilisées physiquement et chimiquement (en particulier contre l'oxydation) et présentées de préférence sous forme stérile. Lorsqu'elles sont destinées à l'alimentation parentérale, elles sont apyrogènes et stériles.

L'invention concerne également un procédé de préparation de corps gras à partir de pépins de fruits du genre Ribes, caractérisé par le fait qu'on moud une matière végétale contenant ces pépins et qu'on l'extrait par solvants de manière à obtenir un corps gras contenant au moins 4% en poids d'acide gamma-linolénique pratiquement dépourvu des composés colorants, des acides gras libres, des colorants et des cires de ces fruits.

Selon l'invention, on entend par matière végétale mise en oeuvre les sous-produits mentionnés ci-dessus. La matière première est généralement sous forme de tourteaux fortement colorés et contenant les cires associées aux pépins, cires et colorants représentant de 5 à 7% en poids de l'huile brute qu'on en extrairait, p. ex. par de l'hexane. Les cires sont des esters saturés et mono-insaturés d'alcools gras et d'acides gras à longue chaîne solides à la température ordinaire. Les cires et colorants en question ne sont pas désirés dans une huile constitutive des compositions nutritives.

Une matière première de choix est le gâteau de pressage des jus de fruits, en particulier de cassis. On le sèche d'abord p. ex. à l'air pendant environ 1 heure à environ 60°C. On le moud grossièrement et on le tamise, de manière à obtenir des particules de 1 à 1,5 mm, le rendement du tamisage étant de 60 à 80% du poids des tourteaux. On peut avantageusement séparer les graines des pailles par gravité dans un courant d'air ou «élutriation» avec un rendement en poids de 80 à 90%.

Selon une forme d'exécution préférée du procédé, on procède au lavage des graines par un solvant polaire d'utilisation courante de manière à les débarrasser des cires, colorants et acides gras libres. Pour les applications cosmétiques, on peut utiliser p. ex. le méthanol, l'isopropanol, l'acétone, l'éthanol ou un mélange de ces solvants ou encore un fluide supercritique p. ex. le dioxyde de carbone dans des conditions lui conférant un caractère polaire. Pour les applications alimentaires et pharmaceutiques on utilisera un solvant autorisé p. ex. l'éthanol ou le dioxyde de carbone supercritique.

A titre d'exemple, le lavage a lieu par extraction des graines par l'éthanol au reflux soit par charges, p. ex. une première fois pendant environ 2 heures et une seconde fois pendant environ 30-60 minutes, soit en continu pendant environ 2 heures puis on essore le résidu.

On peut en variante extraire les graines en utilisant p. ex. le dioxyde de carbone à 250-350 bar et 60-80% en cycle continu, le solvant étant récupéré sous forme gazeuse par diminution de la pression, recomprimé et recyclé. On a constaté que l'extraction permettait d'éliminer la plupart des matières colorantes et des cires présentes dans les pellicules et autour des pépins: en effet, les cires se déposent par refroidissement à partir de l'extrait alcoolique tandis que celui-ci est très fortement coloré.

On moud le résidu essoré (particules 100 à 300 μ), on y ajoute environ 10-15% d'eau puis on le met sous forme de boulettes ou de granules par extrusion de la pâte et découpage du boudin. On peut en variante procéder au floconage des graines sur cylindre aplatisseur à partir du résidu essoré non moulu. Ces mises en forme évitent le colmatage et facilitent l'extraction ultérieure de l'huile en donnant au produit la porosité permettant une bonne pénétration du solvant, et les flocons en particulier résistent à l'écrasement.

L'étape suivante consiste à extraire l'huile avec un solvant non polaire, p. ex. de l'hexane à raison d'environ 250% en poids d'hexane par rapport au résidu. On peut en variante utiliser le dioxyde de carbone liquide ou de préférence supercritique également dans cette étape, dans des conditions où il est faiblement polaire, p. ex. à 200-300 bar et 40-60°C. On peut bien entendu utiliser p. ex. le dioxyde de carbone supercritique dans l'étape de lavage et dans l'étape d'extraction subséquente ou dans une seule de ces étapes, l'autre étant conduite avec p. ex. l'éthanol, respectivement l'hexane.

Après l'extraction, on peut, bien que ses mesures constituent uniquement des options préférées, neutraliser la solution pour supprimer les acides gras libres résiduels, après en avoir évaporé partiellement le solvant, p. ex. dans le cas de l'hexane de sorte qu'elle contienne eviron 10% en poids d'huile, par l'hydroxyde de sodium ou de potassium concentré (2N), la refroidir à environ 0-4°C pendant environ 24 heures et la filtrer à cette température de manière à en éliminer totalement les cires résiduelles. De même, la décoloration et la désodorisation ci-après constituent des options préférées: on décolore la solution par 2-8% en poids de charbon actif par rapport à l'huile engagée à 20°C-60°C puis on évapore l'hexane. On désodorise ensuite l'huile par entraînement à la vapeur à 140-220°C et de préférence à environ 180°C sous un vide égal ou inférieur à 1 torr.

Selon une variante de préparation de l'huile, on procède pas au lavage préalable des graines moulues mais on l'extrait directement par un solvant. Dans ce cas, la neutralisation, l'élimination des cires par décantation, la décoloration et la désodorisation comme indiquées ci-dessus sont indispensables à l'obtention d'une huile raffinée jaune pâle.

Dans certains cas, on désire enrichir le corps gras en acide gamma-linolénique. Pour ce faire on saponifie l'huile débarrassée des cires et des acides gras libres par un hydroxyde alcalin, par exemple l'hydroxyde de potassium en milieu méthanol/eau à la concentration d'environ 11%, on acidifie les sels obtenus par un acide minéral, p. ex. l'acide sulfurique 2N, on extrait les acides gras libérés par l'hexane, on sépare la phase organique et on la sèche p. ex. par addition de sulfate de sodium. On peut en variante traiter directement le tourteau moulu par un hydroxyde alcalin, l'acidifier par un acide minéral, extraire les acides gras libérés à l'hexane et sécher la phase organique comme ci-dessus. On fractionne la phase organique par chromatographie haute performance en phase liquide, par passage sur colonnes de gel de silice chargées avec du nitrate d'argent et élution de préférence avec un mélange de dichlorométhane, toluène et éther diéthylique 70/25/5-65/30/5 en mode isocratique, c'est-à-dire avec recyclage du mélange solvant de composition fixe et on obtient ainsi une fraction contenant environ 60% en poids d'acide gamma-linolénique et environ 40% d'acide alpha-linolénique.

On peut isoler l'acide gamma-linolénique pratiquement pur en effectuant une chromatographie en phase liquide à haute performance avec support coté en $C_8$ ou $C_{18}$ en phase inversée avec gradient de mélange solvant acétonitrile/eau, méthanol/eau ou isopropanol/eau.

Les exemples suivantes illustrent l'invention. Dans ces exemples, les parties et pourcentages sont exprimés en valeur pondérale.

### Exemple 1

On traite 100 kg de résidu obtenu par extraction des jus de cassis et séchage une première fois pendant 2 heures au reflux avec 250 kg d'éthanol et une seconde fois pendant 1 heure au reflux avec 250 kg d'éthanol. On essore et on sèche l'extrait à 80°C pendant 2 fois 30 minutes dans un sécheur à air et on le broie finement dans un broyeur à marteaux.

Après humidification avec 10-15% d'eau et extrusion de la pâte sous forme de boulettes, on extrait les 89 kg de produit obtenu par 2 fois 205 kg d'hexane au reflux pendant 3 heures puis on refroidit et on filtre. On évapore ensuite l'hexane et on obtient 14,3 kg d'une huile jaune clair dont la teneur pondérale en acides gras libres est 0,16%.

### Exemple 2

On broie 100 kg de résidu séché provenant de l'extraction des jus de cassis dans un broyeur à marteaux et on tamise la poudre de façon à obtenir des particules de 1 à 1,5 mm avec un rendement au tamisage de 60-80%. On humidifie et on met le produit sous forme de boulettes et on extrait à l'hexane comme à l'exemple 1. On évapore une partie de l'hexane, on neutralise les acides gras libres par une solution d'hydroxyde de sodium 2N, on sépare la phase organique, on la laisse reposer à 4°C pendant 24 heures et on la sépare des cires dures qui se sont déposées par filtration. On la traite par 2 à 8% de charbon actif basé sur la quantité d'huile en solution puis on évapore le solvant et on désodorise l'huile par entraînement à la vapeur à 180°C sous un vide de 0,1 torr. On obtient ainsi 13 à 16 kg d'huile raffinée.

### Exemple 3

On moud 100 kg de cassis séché provenant du pressage des jus dans un moulin à disques et on tamise le produit moulu de manière à obtenir 60,5 kg de particules comprises entre 1 et 1,5 mm. On l'envoie dans un élutriateur et on recueille 49,5 kg d'une fraction lourde constituée essentiellement de graines. On lave 2 fois avec chaque fois 120 kg d'éthanol au reflux. On procède comme dans l'exemple 1 sauf qu'on utilise 2 fois 120 kg d'éthanol pour le lavage, qu'on met l'extrait séché sous forme de flocons à l'aide d'un broyeur à cylindres aplatisseur au lieu de l'extruder et qu'on extrait les flocons avec 2 fois 102,5 kg d'hexane. On obtient 11,1 kg d'une huile jaune clair.

### Exemples de compositions cosmétiques 4 à 14

| 4 - Crème de soins (émulsion eau dans l'huile) | % en poids |
|---|---|
| Myristate d'isopropyle | 30 |
| Huile de paraffine | 18 |
| Huile de cassis | 10 |
| Ozokérite | 4 |
| Lanolate de magnésium | 14,4 |
| Alcool de lanoline | 3,6 |
| Butyl hydroxy anisole (BHA) + butyl hydroxy toluène (BHT) | 0,01 |
| Eau + conservateur q.s.p. | 100% |

| 5 - Lait corporel | % en poids |
|---|---|
| Huile de vaseline | 8 |
| Huile de cassis | 3 |
| Stéarate de glycérol | 2 |
| Tween 60® [polyoxyéthylène (20) monostéarate de sorbitan] | 1 |

| | |
|---|---|
| Acide stéarique | 1,4 |
| Triéthanolamine | 0,7 |
| Carbopol 940® (neutralisé) | 0,2 |
| BHA + BHT | 0,01 |
| Parfum | 1 |
| Eau + conservateur q.s.p. | 100% |

### 6 - Baume

| | % en poids |
|---|---|
| Ozokérite | 4 |
| Huile de cassis | 12 |
| Miglyol gel | 30 |
| Vaseline | 20 |
| Huile de soja | 15 |
| Huile de tournesol | 19 |

### 7 - Huile pour le corps

| | % en poids |
|---|---|
| Huile de cassis | 30 |
| Huile de soja | 10 |
| Huile de tournesol | 30 |
| Huile d'arachide | 29,8 |
| BHA + BHT | 0,2 |

### 8 - Masque

| | % en poids |
|---|---|
| Huile de cassis | 10 |
| Alcool cétylique | 3 |
| Alcool stéarylique | 3 |
| Alcool gras polyoxyéthyléné (Polysorbate 80®) | 4 |
| Propylène glycol-1,2 | 5 |
| Glycérine | 2 |
| Dioxyde de titane | 3,5 |
| Conservateur | 0,3 |
| Eau distillée, concentré de parfum, antioxydant (BHA + BHT) q.s.p. | 100% |

On peut y ajouter selon désir des extraits de plantes ou des extraits biologiques.

### 9 - Crème à raser

| | % en poids |
|---|---|
| Huiles de cassis | 9,4 |
| Acide stéarique | 7 |
| Alcool cétylique | 0,7 |
| Monostéarate de poly-éthylène glycol | 3,5 |
| BHA + BHT | 0,05 |
| Glycérol | 9 |
| Conservateur | 0,3 |
| Triéthanolamine | 2,7 |
| Parfum et eau q.s.p. | 100% |

### 10 - Fard à paupières

| | % en poids |
|---|---|
| Talc | 50 |
| Amidon de blé | 12 |
| Stéarate de zinc | 3 |
| Bleu d'outremer | 4,5 |
| Oxyde de fer jaune | 3,2 |
| Oxyde de fer noir | 0,5 |
| Oxyde de fer brun | 0,8 |
| Oxyde de chrome | 2,5 |
| Mica titane | 10 |
| Mica titane + oxyde de fer | 3,5 |
| Agglomérant | 10 |

| Formule de l'agglomérant: | % en poids |
|---|---|
| Huile de vaseline | 50 |
| Lanoline liquide | 20 |
| Huile de cassis | 20 |
| Monostéarate de glycérol | 9 |
| Parahydroxy benzoate de propyle | 0,5 |
| BHA + BHT | 0,5 |

### 11 - Rouge à lèvres

| | % en poids |
|---|---|
| *Corps blanc:* | |
| Cire microcristalline | 12 |
| Cire de Candelilla | 2 |
| Esters lourds de synthèse (Synchro wax ERLC® de Croda) | 4 |
| Huile de ricin | 10 |
| Huile de cassis | 15 |
| Lanolate d'isopropyle | 15 |
| Lanoline liquide | 10 |
| Lanoline acétylée | 12 |
| Vaseline | 10 |
| Ricinoléate de cétyle | 9,8 |
| BHA + BHT (50/50) | 0,2 |

auquel on ajoute 5,4 parties en poids des pigments suivants pour 100 parties en poids de corps blanc:

| *Pigments minéraux et organiques:* | |
|---|---|
| Oxyde de fer rouge | 1,2 |
| Oxyde de titane | 1,3 |
| D et C red No 9 | 2 |
| D et C red No 27 | 0,4 |
| Parfum | 0,5 |

### 12 - Fond de teint (émulsion eau dans l'huile)

| | % en poids |
|---|---|
| *Phase grasse:* | |
| Huile de paraffine | 5 |
| Huile de cassis | 5 |
| Graisse de shorea | 4 |
| Perhydrosqualène | 6 |
| Ozokérite | 2 |
| Lanolate de magnésium | 5 |
| Alcool de lanoline | 3 |
| Oxyde de fer | 3 |
| Dioxyde de titane | 4 |
| Polyéthylène | 10 |
| Parfum | 0,4 |
| BHA + BHT | 0,5 |
| Eau + conservateur q.p.s. | 100% |

### 13 - Huile de bain pour bébé

| | % en poids |
|---|---|
| Huile de cassis | 20 |
| Ether polyglycolique d'alcool oléique | 15 |
| Triglycérides à chaîne moyenne $(C_8-C_{10})$ | 15 |
| Myristate d'isopropyle | 30 |
| Palmitate d'octyle | 10 |
| Antioxydant (BHA + BHT) et parfum q.s.p. | 100% |

**14 - *Traitement capillaire à rincer***    *% en poids*

On prépare la composition suivante:

| | |
|---|---|
| Alcool stéarylique | 1,8 |
| Alcool cétylstéarylique à 15 moles O.E. | 5,6 |
| Huile de cassis | 2,5 |
| Cellulose quaternisé vendue sous la dénomination JR 400 par la société Union Carbide | 0,8 |
| Eau q.s.p. | 100,0 |

On applique cette composition sur des cheveux propres. Après 5 min de pose, on rince.

Les cheveux mouillés se démêlent facilement et la chevelure propre est douce et soyeuse.

*Exemples d'aliments diététiques 15 à 17*

**15 - *Sauce pour salade***    *% en poids*

| | |
|---|---|
| Huile de cassis | 10 |
| Huile de pépins de raisin | 14,5 |
| Vinaigre blanc | 3,2 |
| Vinaigre rouge | 4,4 |
| Poudre de moutarde | 5,35 |
| Poudre d'oignon | 0,25 |
| Sel | 1,4 |
| Sucre | 1 |
| Agent émulsionnant | 2,5 |
| Palmitate d'ascorbyle | 0,1 |
| Poudre d'oeuf | 4,2 |
| Eau | 53,1 |

**16 - *Huile pour salade***    *% en poids*

| | |
|---|---|
| Huile de cassis | 10 |
| Huile de pépins de raisin | 90 |

**17 - *Lait infantile***    *Parties en poids*

| | |
|---|---|
| Huile de cassis (correspond à 0,35% d'acide gamma-linolénique calculé sur le poids total des graisses, ce qui est le taux du lait humain) } | 0,61 |
| Huile de maïs | 2,47 |
| Graisse lactique | 11,64 |
| Triglycérides à chaîne moyenne | 9,28 |
| Hydrates de carbon dont 41,6 de lactose et 15,3 de glucose | 56,9 |
| Protéines dont 4,4 de caséine et 10 de protéine de lactosérum | 14,4 |
| Cendres dont | 1,7 |
| Calcium | $350 . 10^{-3}$ |
| Phosphore | $200 . 10^{-3}$ |
| Potassium | $370 . 10^{-3}$ |
| Sodium | $103 . 10^{-3}$ |
| Eau | 3,0 |

ainsi que les quantités minimales des vitamines et oligo-éléments ci-après:

| | |
|---|---|
| Fer | $6,0 . 10^{-3}$ |
| Cuivre | $0,3 . 10^{-3}$ |
| Zinc | $1,8 . 10^{-3}$ |
| Iode | $25 . 10^{-6}$ |
| Acide folique | $80 . 10^{-6}$ |
| Vitamine C | $200 . 10^{-3}$ |
| Vitamine E | $10 . 10^{-3}$ |
| Vitamina $B_1$ | $0,35 . 10^{-3}$ |
| Vitamina $B_2$ | $0,5 . 10^{-3}$ |
| Vitamina $B_6$ | $0,9 . 10^{-3}$ |
| Vitamina $B_{12}$ | $1,7 . 10^{-3}$ |
| Vitamina pp | $5,2 . 10^{-3}$ |
| Pantothénate de calcium | $2,6 . 10^{-3}$ |

Une telle poudre est préparée p. ex. comme décrit dans le brevet français No. 2 388 503.

*Exemples de compositions pharmaceutiques 18 à 19*

**18 - *Capsules pour administration orale***

On prépare des capsules de gélatine contenant 500 mg d'huile de cassis correspondant à 80 mg d'acide gamma-linolénique par capsule.

**19 - *Sirop de fruits à usage interne***    *% en poids*

| | |
|---|---|
| Huile de cassis | 25 |
| Mélange de mono- et diglycérides | 2 |
| Mélange de gommes | 0,7 |
| Sucre | 17 |
| Mélange naturel de tocophérols ($\alpha$, $\beta$, $\gamma$ et $\delta$) | 0,1 |
| Conservateur, arômes et eau q.s.p. | 100% |

**Revendications**

1. Composition nutritive, caractérisée par le fait qu'elle contient 1 à 80% en poids d'un corps gras extrait à partir des pépins de fruits du genre Ribes contenant au moins 4% d'acide gamma-linolénique pratiquement dépourvu des composés odorants, des acides gras libres, des colorants et des cires de ces fruits.

2. Composition nutritive selon la revendication 1, caractérisée par le fait que le corps gras est l'huile de cassis.

3. Composition cosmétique ou dermatologique selon la revendication 1 aqueuse ou anhydre contenante une phase grasse, caractérisée par le fait que la phase grasse contient 1 à 80% en poids d'un corps gras extrait à partir des pépins de fruits du genre Ribes.

4. Aliment diététique ou de supplémentation selon la revendication 1 comportant de la matière grasse, caractérisé par le fait que la matière grasse est constituée de 2 à 15% en poids d'un corps gras extrait à partir de pépins de fruits du genre Ribes.

5. Composition pharmaceutique selon la revendication 1, sous une forme adaptée à l'administration topique, orale, entérale ou parentérale, caractérisée par le fait qu'elle contient de 5 à 80% en poids d'un corps gras extrait à partir de pépins de fruits du genre Ribes.

6. Procédé de préparation de corps gras à partir de pépins de fruits du genre Ribes caractérisé par le fait qu'on moud une matière végétale contenant ces pépins et qu'on l'extrait par solvants de manière à obtenir un corps gras contenant au moins 4% en poids d'acide gamma-linolénique pratiquement dépourvu des composés odorants, des acides gras libres, des colorants et des cires de ces fruits.

7. Procédé selon la revendication 6, caractérisé par le fait que la matière végétale mise en oeuvre est constituée de résidus de la production des jus, des gelées ou confitures, des eaux-de-vie, liqueurs ou schnaps de cassis, de groseilles, de groseilles à maquereau, d'hybrides ou de mélanges de ces fruits.

8. Procédé selon la revendication 6, caractérisé par le fait qu'on moud la matière végétale préalablement séchée, qu'on lave la matière végétale moulue avec un solvant polaire ou un fluide supercritique à l'état polaire de manière à la débarrasser des cires, de la plupart des matières colorantes et des acides gras libres, qu'on l'essore, qu'on moud finement le résidu, qu'on l'humidifie et qu'on le met sous forme de granules ou de boulettes, qu'on l'extrait par un solvant non polaire ou un fluide supercritique à l'état faiblement polaire et qu'on élimine ce dernier par évaporation.

9. Procédé selon la revendication 6, caractérisé par le fait qu'on moud la matière végétale préalablement séchée, qu'on lave la matière végétale moulue avec un solvant polaire ou un fluide supercritique à l'état polaire de manière à la débarrasser des cires, de la plupart des matières colorantes et des acides gras libres, qu'on l'essore, qu'on soumet le résidu essoré à un tamisage et à une élutriation de manière qu'il soit essentiellement constitué de pépins de 1 à 1,5 mm et qu'on met les pépins sous forme de flocons, qu'on extrait les flocons par un solvant non polaire ou un fluide supercritique à l'état faiblement polaire et qu'on élimine ce dernier par évaporation.

10. Procédé selon la revendication 8 ou 9, caractérisé par le fait qu'on lave la matière végétale moulue avec l'éthanol au reflux.

11. Procédé selon la revendication 8 ou 9, caractérisé par le fait qu'on lave la matière végétale avec le dioxyde de carbone supercritique à l'état polaire sous une pression supérieure à 250 bar et à une température de 60 à 80°C.

12. Procédé selon la revendication 8 ou 9, caractérisé par le fait qu'on extrait le résidu lavé par l'hexane au reflux.

13. Procédé selon la revendication 8 ou 9, caractérisé par le fait qu'on extrait le résidu lavé par le dioxyde de carbone supercritique à l'état faiblement polaire sous une pression de 200 à 300 bar et à une température de 40 à 60°C.

14. Procédé selon la revendication 8, caractérisé par le fait qu'après l'extraction du résidu, on évapore le solvant non polaire partiellement jusqu'à obtenir une solution contenant 80 à 90% en poids de solvant, on neutralise la solution par un alcali concentré, on maintient la solution à une température de 0 à 4°C pendant environ 24 h, on la filtre et on élimine le solvant par évaporation.

15. Procédé selon la revendication 14. caractérisé par le fait qu'après extraction du résidu, évaporation partielle du solvant et neutralisation de la solution, on la décolore par 2 à 8% de charbon actif basé sur le poids du corps gras en solution.

16. Procédé selon l'une des revendications 14 et 15, caractérisé par le fait qu'après évaporation du solvant, on désodorise l'huile à une température de 140-220°C et sous un vide inférieur à 1 torr.

17. Procédé selon la revendication 8, caractérisé par le fait qu'un moud la matière végétale préalablement séchée et qu'on la tamise, qu'on la met sous forme de flocons ou qu'on la moud finement, qu'on l'humidifie et qu'on la met sous forme de granules ou de boulettes, qu'on l'extrait par un solvant non polaire, qu'on évapore le solvant partiellement jusqu'à obtenir une solution contenant 80 à 90% en poids de solvant, qu'on neutralise la solution par l'hydroxyde de sodium ou de potassium concentré, qu'on maintient la solution à une température d'environ 4°C pendant environ 24 h, qu'on la filtre, qu'on la décolore par 2 à 8% de charbon actif basé sur le poids de l'huile en solution, qu'on évapore le solvant et qu'on désodorise l'huile à une température de 140-220°C et sous un vide inférieur à 1 torr.

18. Procédé selon la revendication 17, caractérisé par le fait qu'on saponifie l'huile obtenue par un hydroxyde alcalin en milieu hydroalcoolique, qu'on acidifie les sels obtenus par un acide minéral concentré, qu'on extrait les acides gras libérés par addition de solvant non polaire, séparation et séchage de la phase organique et qu'on sépare une fraction enrichie en acide gamma-linolénique par chromatographie haute performance en phase liquide sur colonnes de gel de silice chargées en cations argent en mode isocratique.

19. Procédé selon la revendication 8, caractérisé par le fait qu'on traite la matière végétale humide moulue par un hydroxyde alcalin, qu'on acidifie la suspension par un acide minérale concentré, qu'on extrait les acides gras libérés par addition d'un solvant non polaire, séparation et séchage de la phase organique et qu'on sépare une fraction enrichie en acide gamma-linolénique par chromatographie haute performance en phase liquide sur colonnes de gel de silice chargées de nitrate d'argent en mode isocratique.

20. Procédé selon la revendication 18 ou 19, caractérisé par le fait qu'en utilisant la chromatographie liquide à haute performance avec support coté en $C_8$ ou $C_{18}$ en phase inversée et un gradient de solvants, on élue une fraction contenant essentiellement l'acide gamma-linolénique.

**Patentansprüche**

1. Nahrhafte Zusammensetzung, dadurch gekennzeichnet, dass sie 1 bis 80 Gew.-% eines aus den Kernen von Früchten der Gattung Ribes extrahierten Fettkörpers enthält, der wenigstens 4% γ-Linolensäure enthält und praktisch frei von Geruchsverbindungen, freien Fettsäuren, Farbstoffen und Wachsen dieser Früchte ist.

2. Nahrhafte Zusammensetzung nach Anspruch 1, dadurch gekennzeichnet, dass der Fettkörper das Cassisöl ist.

3. Eine Fettphase enthaltende wässerige oder wasserfreie kosmetische oder dermatologische Zusammensetzung nach Anspruch 1, dadurch gekennzeichnet, dass die Fettphase 1 bis 80 Gew.-% eines aus den Kernen von Früchten der Gattung Ribes extrahierten Fettkörpers enthält.

4. Fetthaltiges diätetisches oder Ergänzungsnahrungsmittel nach Anspruch 1, dadurch gekennzeichnet, dass das Fett aus 2 bis 15 Gew.-% eines aus den Kernen von Früchten der Gattung Ribes extrahierten Fettkörpers besteht.

5. Zur topischen, oralen, enteralen oder parenteralen Anwendung geeignete pharmazeutische Zusammensetzung nach Anspruch 1, dadurch gekennzeichnet, dass sie 5 bis 80 Gew.-% eines aus den Kernen von Früchten der Gattung Ribes extrahierten Fettkörpers enthält.

6. Verfahren zur Herstellung eines Fettkörpers aus den Kernen von Früchten der Gattung Ribes, dadurch gekennzeichnet, dass man ein diese Kerne enthaltendes Pflanzenmaterial mahlt und es mit Lösungsmitteln derart extrahiert, dass man einen Fettkörper erhält, der wenigstens 4 Gew.-% γ-Linolensäure enthält und der praktisch frei von Geruchsverbindungen, freien Fettsäuren, Farbstoffen und Wachsen dieser Früchte ist.

7. Verfahren nach Anspruch 6, dadurch gekennzeichnet, dass das eingesetzte Pflanzenmaterial aus Rückständen der Herstellung von Säften, Gelees oder Marmeladen, von Branntweinen, Likören oder Schnaps von schwarzen Johannisbeeren (Cassis), Johannisbeeren, Stachelbeeren, von Hybriden oder von Gemischen dieser Früchte besteht.

8. Verfahren nach Anspruch 6, dadurch gekennzeichnet, dass man das zuvor getrocknete Pflanzenmaterial mahlt, das gemahlene Pflanzenmaterial mit einem polaren Lösungsmittel oder einem überkritischen Fluid in polarem Zustand wäscht, um es von Wachsen, dem Grossteil der farbgebenden Substanzen und den freien Fettsäuren zu befreien, dass man es trocknet, den Rückstand fein vermahlt, diesen befeuchtet und in Granulat- oder Pelletsform überführt, mit einem nicht-polaren Lösungsmittel oder einem schwach polaren überkritischen Fluid extrahiert und dass man das letztgenannte durch Verdampfen beseitigt.

9. Verfahren nach Anspruch 6, dadurch gekennzeichnet, dass man das zuvor getrocknete Pflanzenmaterial mahlt, das gemahlene Pflanzenmaterial mit einem polaren Lösungsmittel oder einem in polarem Zustand vorliegenden überkritischen Fluid wäscht, um es von den Wachsen, dem Grossteil der farbgebenden Substanzen und den freien Fettsäuren zu befreien, dass man es trocknet, den getrockneten Rückstand einer Siebung und einer Schwerkrafttrennung unterwirft, so dass dieser im wesentlichen aus Kernen von 1 bis 1,5 mm besteht, und die Kerne in Flockenform überführt und die Flocken mit einem nicht-polaren Lösungsmittel oder einem in schwach polarem Zustand vorliegenden überkritischen Fluid extrahiert und das letztgenannte durch Verdampfen beseitigt.

10. Verfahren nach Anspruch 8 oder 9, dadurch gekennzeichnet, dass man das gemahlene Pflanzenmaterial mit Ethanol am Rückfluss wäscht.

11. Verfahren nach Anspruch 8 oder 9, dadurch gekennzeichnet, dass man das gemahlene Pflanzenmaterial mit überkritischem Kohlendioxid in polarem Zustand bei einem Druck oberhalb von 250 bar und bei Temperatur von 60 bis 80°C wäscht.

12. Verfahren nach Anspruch 8 oder 9, dadurch gekennzeichnet, dass man den gewaschenen Rückstand mit Hexan unter Rückfluss extrahiert.

13. Verfahren nach Anspruch 8 oder 9, dadurch gekennzeichnet, dass man den gewaschenen Rückstand mit überkritischem Kohlendioxid in schwach polarem Zustand bei einem Druck von 200 bis 300 bar und bei einer Temperatur von 40 bis 60°C extrahiert.

14. Verfahren nach Anspruch 8, dadurch gekennzeichnet, dass man nach der Extraktion des Rückstandes das nicht-polare Lösungsmittel partiell verdampft, bis man eine 80 bis 90 Gew.-% Lösungsmittel enthaltende Lösung erhält, die Lösung mit einem konzentrierten Alkali neutralisiert, die Lösung etwa 24 Stunden lang bei einer Temperatur von 0 bis 4°C hält, sie filtriert und das Lösungsmittel durch Verdampfen beseitigt.

15. Verfahren nach Anspruch 14, dadurch gekennzeichnet, dass man nach der Extraktion des Rückstandes, partieller Verdampfung des Lösungsmittels und Neutralisation der Lösung diese mit 2 bis 8% Aktivkohle, bezogen auf das Gewicht des gelösten Fettkörpers, entfärbt.

16. Verfahren nach einem der Ansprüche 14 und 15, dadurch gekennzeichnet, dass man nach Verdampfung des Lösungsmittels das Öl bei einer Temperatur von 140 bis 220°C und bei einem Vakuum von unter 1 Torr desodorisiert.

17. Verfahren nach Anspruch 8, dadurch gekennzeichnet, dass man das zuvor getrocknete Pfalnzenmaterial mahlt und es siebt, es in Flockenform bringt oder fein mahlt, es befeuchtet und in Granulat- oder Pelletsform überführt, es mit einem nicht-polaren Lösungsmittel extrahiert, das Lösungsmittel partiell verdampft, bis man eine 80 bis 90 Gew.-% Lösungsmittel enthaltende Lösung erhält, die Lösung mit konzentriertem Natrium- oder Kaliumhydroxid neutralisiert, die Lösung etwa 24 Stunden lang bei einer Temperatur von ungefähr 4°C hält, sie filtriert, sie mit 2 bis 8% Aktivkohle, bezogen auf das Gewicht des in Lösung befindlichen Öls, entfärbt, das Lösungsmittel verdampft und das Öl bei einer Temperatur von 140 bis 220°C und unter einem Vakuum von weniger als 1 Torr desodorisiert.

18. Verfahren nach Anspruch 17, dadurch gekennzeichnet, dass man das erhaltene Öl in wässerigalkoholischem Milieu mit einem Alkalihydroxid verseift, die erhaltenen Salze mit einer konzentrierten Mineralsäure ansäuert, die freigesetzten Fettsäuren durch Zugabe von nicht-polarem Lösungsmittel, Trennung und Trocknung der organischen Phase extrahiert und eine an γ-Linolensäure angereicherte Fraktion durch isokratische Hochleistung-Flüssigkeitschromatografie an mit Silberkationen beladenen Kieselgelsäulen abtrennt.

19. Verfahren nach Anspruch 8, dadurch gekennzeichnet, dass man das feuchte, gemahlene Pflanzenmaterial mit einem Alkalihydroxid behandelt, die Suspension mit einer konzentrierten Mineralsäure ansäuert, die freigesetzten Fettsäuren durch Zugabe eines nicht-polaren Lösungsmittels, Trennung und Trocknung der organischen Phase extrahiert und eine an $\gamma$-Linolensäure angereicherte Fraktion durch isokratische Hochleistung-Flüssigkeitschromatografie an mit Sibernitrat beladenen Kieselgelsäulen abtrennt.

20. Verfahren nach Anspruch 18 oder 19, dadurch gekennzeichnet, dass man bei Anwendung der Hochleistung-Flüssigkeitschromatografie mit einem mit $C_8$ oder $C_{18}$ beschichteten Träger in inverser Phase und mit einem Lösungsmittelgradienten eine Fraktion eluiert, die im wesentlichen die $\gamma$-Linolensäure enthält.

## Claims

1. A nutritive composition containing from 1 to 80% by weight of a fatty substance extracted from pips of fruits of the <u>Ribes</u> genus containing at least 4% of $\gamma$-linolenic acid which is substantially free from the odorous compounds, the free fatty acids, the colourings and the waxes of these fruits.

2. A nutritive composition according to claim 1, wherein the fatty substance is blackcurrant oil.

3. A composition as claimed in claim 1, which is an aqueous or anhydrous cosmetic or dermatological composition containing an oil phase, wherein the oil phase contains from 1 to 80% by weight of the fatty substance extracted from pips of fruits of the Ribes genus.

4. A composition as claimed in claim 1, which is a dietetic food or food supplement comprising a fatty material, wherein the fatty material comprises from 2 to 15% by weight of the fatty substance extracted from pips of fruits of the <u>Ribes</u> genus.

5. A composition as claimed in claim 1, which is a pharmaceutical composition containing from 5 to 80% by weight of the fatty substance extracted from pips of fruits of the <u>Ribes</u> genus, in a form adapted for topical, oral, enteral or parenteral administration.

6. A process for the preparation of fatty substances from pips of fruits of the <u>Ribes</u> genus, in which a vegetable material containing such pips is ground and extracted with a solvent in order to obtain a fatty substance containing at least 4% by weight of $\gamma$-linolenic acid which is substantially free from the odorous compounds, the free fatty acids, the colourings and the waxes of these fruits.

7. A process as claimed in claim 6, wherein the vetable material which is used comprises residues from the production of juices, jellies, or preserves, brandies, liqueurs, or schnapps of blackcurrants, redcurrants, gooseberries, hybrids or mixtures of these fruits.

8. A process as claimed in claim 6 or 7, wherein the vegetable material which has been previously dried is ground, the ground vetable material is washed with a polar solvent or a supercritical fluid in a polar condition in order to remove the waxes, most of the colouring materials and the free fatty acids, the material is drained, the residue is finely ground, moistened and made into granules or pellets, extracted with a non-polar solvent or a supercritical fluid in a slightly polar condition and the latter is removed by evaporation.

9. A process as claimed in claim 6 or 7, wherein the vegetable material which has been previously dried is ground, the ground vegetable material is washed with a polar solvent or a supercritical fluid in a polar condition in order to remove the waxes, most of the colouring materials and the free fatty acids, the material is drained, the drained residue is subjected to a sieving operation and an elutriation operation so that it essentially comprises pips of from 1 to 1.5 mm, and the pips are made into flakes, the flakes are extracted with a non-polar solvent or a supercritical fluid in a slightly polar condition and the latter is removed by evaporation.

10. A process as claimed in claim 8 or 9, wherein the ground vegetable material is washed with ethanol under reflux.

11. A process as claimed in claim 8 or 9, wherein the ground vegetable material is washed with supercritical carbon dioxide in a polar condition under a pressure of above 250 bars and at a temperature of from 60 to 80°C.

12. A process as claimed in claim 8 or 9, wherein the washed residue is extracted with hexane under reflux.

13. A process as claimed in claim 8 or 9, wherein the washed residue is extracted with supercritical carbon dioxide in a slightly polar condition under a pressure of from 200 to 300 bars and at a temperature of from 40 to 60°C.

14. A process as claimed in claim 8, wherein after the residue has been extracted, the non-polar solvent is partally evaporated until a solution containing from 80 to 90% by weight of solvent is obtained, the solution is neutralized with a concentrated alkali, is maintained at a temperature of from 0 to 4°C for about 24 hours, is filtered, and the solvent is removed by evaporation.

15. A process as claimed in claim 14, wherein after the residue has been extracted, the solvent has been partially evaporated and the solution has been neutralized, the solution is decolorized using from 2 to 8% of active carbon based on the weight of the fatty substance in solution.

16. A process as claimed in claim 14 or 15, wherein after the solvent has been evaporated, the oil is deodorized at a temperature of from 140 to 220°C and under a vacuum of less than 1 torr.

17. A process as claimed in claim 8, wherein the vegetable material which has been previously dried is ground and sieved, made into flakes or finely ground, moistened and made into granules or pellets, extracted with a non-polar solvent, the solvent is partially evaporated until a solution containing from 80 to 90% by weight of solvent is obtained, the solution is neutralized with concentrated sodium or potassium hydroxide, is maintained at a temperature of about 4°C for about 24 hours, filtered, decolorized with from 2 to 8% of active carbon based on the weight of the oil in solution, the solvent is evaporated and the oil is deodorized at a a temperature of from 140 to 220°C and under a vacuum of less than 1 torr.

18. A process as claimed in claim 17, wherein the oil which is obtained is saponified with an alkali metal hydroxide in a water-alcohol medium, the resulting salts are acidified with a concentrated mineral acid, the free fatty acids are extracted by the addition of a non-polar solvent, by separating and drying the organic phase and a fraction concentrated in $\gamma$-linolenic acid is separated by high performance chromatography in liquid phase over a silica gel column charged with silver cations by an isocratic method.

19. A process as claimed in claim 8, wherein the moist, ground vegetable material is treated with an alkali metal hydroxide, the suspension is acidified with a concentrated mineral acid, the free fatty acids are extracted by the addition of a non-polar solvent, by separating and drying the organic phase, and a fraction concentrated in $\gamma$-linolenic acid is separated by high performance chromatography in liquid phase over a silica gel column charged with silver nitrate by an isocratic method.

20. A process as claimed in claim 18 or 19, wherein by using high performance liquid phase chromatography with a $C_8$ or $C_{18}$ support in reversed phase and by using a solvent gradient, a fraction essentially containing $\gamma$-linolenic acid is eluted.